# EUROPEAN PATENT SPECIFICATION

(11) **EP 3 492 009 B1**
(45) Date of publication and mention of the grant of the patent: **13.12.2023**
(21) Application number: 17204514.8
(22) Date of filing: 29.11.2017
(51) Int. Cl.: A61B 5/00, A61B 5/053, A61B 5/282, A61B 5/113

(54) **AN APPARATUS COMPRISING A FABRIC SUBSTRATE AND ELECTRODES**
VORRICHTUNG MIT EINEM GEWEBESUBSTRAT UND ELEKTRODEN
APPAREIL COMPRENANT UN SUBSTRAT DE TISSU ET DES ÉLECTRODES

(43) Date of publication of application: 05.06.2019
(73) Proprietor: Nokia Technologies Oy, 02610 Espoo (FI)
(72) Inventor: Blomqvist, Kim, 26250 Espoo (FI); Lasarov, Harri, 02330 Espoo (FI)
(74) Representative: Swindell & Pearson Limited

(56) References cited:
- WO-A2-2007/126435
- US-A- 4 122 843
- US-A1- 2006 111 640
- US-A1- 2007 083 096
- US-A1- 2010 113 910
- US-A1- 2010 324 404
- US-A1- 2017 303 808
- Texas Instruments: "microPower, Single-Supply, CMOS Instrumentation Amplifier", , 1 February 2006 (2006-02-01), XP055450956, Retrieved from the Internet: URL:http://www.ti.com/lit/ds/sbos168d/sbos 168d.pdf [retrieved on 2018-02-14]

## Description

### TECHNOLOGICAL FIELD

Embodiments of the present invention relate to an apparatus comprising a fabric substrate and at least one sensing electrode.

### BACKGROUND

A sensing electrode may be placed adjacent a portion of a subject's body to sense a bio signal from the subject's body. This may, for example, be useful in impedance tomography, sensing of electrocardiograms (ECG) or in other measurements of electrical signals and/or impedance.

In some circumstances, the anxiety induced by taking measurements in a clinical situation can change measurements.

Also it may be uncomfortable or inconvenient for a subject when measurements need to be taken over an extended period of time.

It would therefore be desirable to be able to take measurements using an electrode outside of a clinical environment and/or over an extended period of time in a manner that is comfortable to the subject.

US4122843 (D1) relates to an electrode system for a heart rate monitor. According to D1, two signal electrodes and a reference electrode are attached to dielectric members, from which their front faces project for engaging a person's skin. According to D1, embedded in the dielectric members that enclose the two signal electrodes are electrostatic shields that are behind the electrodes and spaced from them. According to D1, there are electrical conductors for connecting the signal electrodes to the differential amplifier of a heart rate monitor, and other electrical conductors for connecting the shields and the reference electrode to the common input terminal of the amplifier.

US2010/113910 (D6) relates to a sensor arrangement with at least one sensor and a method for monitoring physiological parameters of a person, a textile fabric and a use of a textile fabric. According to D6, a sensor arrangement is described that is suited to improve signal quality and suppress noise, for instance in remote capacitive sensing of body parameters. According to D6, to achieve this, certain textile fabrics are used, preferably integrated into textile used in a bed, e.g. the blanket, the bed cover, or the mattress. According to D6, these textile fabrics allow for a suppression of electromagnetic interference from external sources and can be arranged to avoid charge build-up during measurements, in particular those caused by movements of the person.

### BRIEF SUMMARY

According to the claimed invention there is provided an apparatus comprising: a fabric substrate; at least one sensing electrode at an interior surface of the fabric substrate for placement adjacent a first portion of a subject's body; at least one reference electrode at the interior surface of the fabric substrate for placement adjacent a second portion of a subject's body; and a first conductive interconnect connecting to the at least one reference electrode wherein the first conductive interconnect is arranged and positioned on a portion of the fabric substrate other than the interior surface of the fabric substrate to provide part of a shield to the at least one sensing electrode.

In some, but not necessarily all examples, the first conductive interconnect is a fabric conductive-interconnect at a first surface of the fabric substrate that is not an interior surface of the fabric substrate.

In some, but not necessarily all examples, the at least one sensing electrode is a fabric electrode at a second surface, the interior surface, of the fabric substrate and/or
the at least one reference electrode is a fabric electrode at a second surface, the interior surface, of the fabric substrate.

In some, but not necessarily all examples, the apparatus comprises at least one second conductive interconnect connecting to the at least one sensing electrode, wherein each at least one second conductive interconnect is a fabric conductive-interconnect.

In some, but not necessarily all examples, the apparatus comprises flexible circuitry operatively interconnected to the at least one sensing electrode and the at least one reference electrode for producing a sensing signal.

In some, but not necessarily all examples, the apparatus is configured as a chest strap configured to position the at least one sensing electrode in contact with a front or side portion of a subject's thorax and to position the at least one reference electrode in contact with a rear portion of a subject's thorax. In some, but not necessarily all examples, the chest strap is configured to maximize a distance of separation between the at least one sensing electrode and the at least one reference electrode.

In some, but not necessarily all examples, the apparatus comprises: a first sensing electrode at a position on an interior surface of the fabric substrate for placement adjacent a portion of a subject's body; a second sensing electrode at a different position on the interior surface of the fabric substrate for placement adjacent a different portion of a subject's body; circuitry configured to provide a common mode voltage at the reference electrode.

In some, but not necessarily all examples, the apparatus comprises: a first sensing electrode at a position on an interior surface of the fabric substrate for placement adjacent a portion of a subject's body; a second sensing electrode at a different position on the interior surface of the fabric substrate for placement adjacent a different portion of a subject's body; ECG circuitry configured to provide an ECG signal from the first sensing electrode and the second sensing electrode; input circuitry configured to apply a time-varying input signal at the first sensing electrode; and output circuitry configured to detect a time-varying output signal at the second sensing electrode.

In some, but not necessarily all examples, the apparatus is configured to tension the fabric substrate in use, the fabric substrate comprising at least a fabric conductive-interconnect configured to resiliently change impedance with a change in tension during use. In some, but not necessarily all examples, an electric path between input circuitry and output circuitry comprises the at least one fabric conductive-interconnect configured to resiliently change impedance with a change in tension during use. In some, but not necessarily all examples, the fabric conductive-interconnect comprises a resiliently extendible filament stitched into the fabric substrate. In some, but not necessarily all examples, the fabric conductive-interconnect comprises a resiliently extendible filament stitched into the fabric substrate so that it extends along a surface of the fabric substrate in a series of alternating curves.

In some, but not necessarily all examples, the apparatus is clothing, wherein the fabric substrate is part of the clothing.

According to various, but not necessarily all, embodiments of the invention there is provided a method comprising: providing a fabric substrate; providing at least one sensing electrode at an interior surface of the fabric substrate for placement adjacent a first portion of a subject's body; providing at least one reference electrode at the interior surface of the fabric substrate for placement adjacent a second portion of a subject's body; and providing a first conductive interconnect connecting to the at least one reference electrode wherein the first conductive interconnect is arranged and positioned on a portion of the fabric substrate other than the interior surface of the fabric substrate to provide part of a shield to the at least one sensing electrode.

According to various, but not necessarily all, embodiments of the invention there is provided examples as claimed in the appended claims.

The scope of protection sought for various embodiments of the invention is set out by the independent claims. The embodiments and features, if any described in this specification that do not fall under the scope of the independent claims are to be interpreted as examples useful for understanding various embodiments of the invention.

### BRIEF DESCRIPTION

For a better understanding of various examples that are useful for understanding the detailed description, reference will now be made by way of example only to the accompanying drawings in which:
Fig. 1 illustrates an example of an apparatus comprising a fabric substrate and electrodes;
Figs 2A, 2B, 2C illustrates examples of fabric substrates;
Fig. 3 illustrates an example of an apparatus comprising a fabric substrate with fabric electrodes and fabric interconnects;
Fig. 4A illustrates a plan view of an example of the interior surface of the fabric substrate illustrated in Fig 3 and Fig. 4C illustrates a plan view of the exterior surface of the fabric substrate illustrated in Fig 3;
Fig 5 illustrates an example of a reference electrode;
Fig. 6 illustrates an example of the apparatus comprising circuitry;
Fig. 7 illustrates an example of the apparatus comprising electrocardiograph (ECG) circuitry, and signal-source circuitry and signal receive circuitry;
Fig. 8A illustrates a perspective view of the apparatus configured as a strap and Fig. 8B illustrates a view of the apparatus configured as a strap from a plan view;
Fig. 9 illustrates an example of a suitable fabric conductive interconnect configured to cause a change in impedance with a change in tension during use; and
Fig 10 illustrates an example of a method.

### DETAILED DESCRIPTION

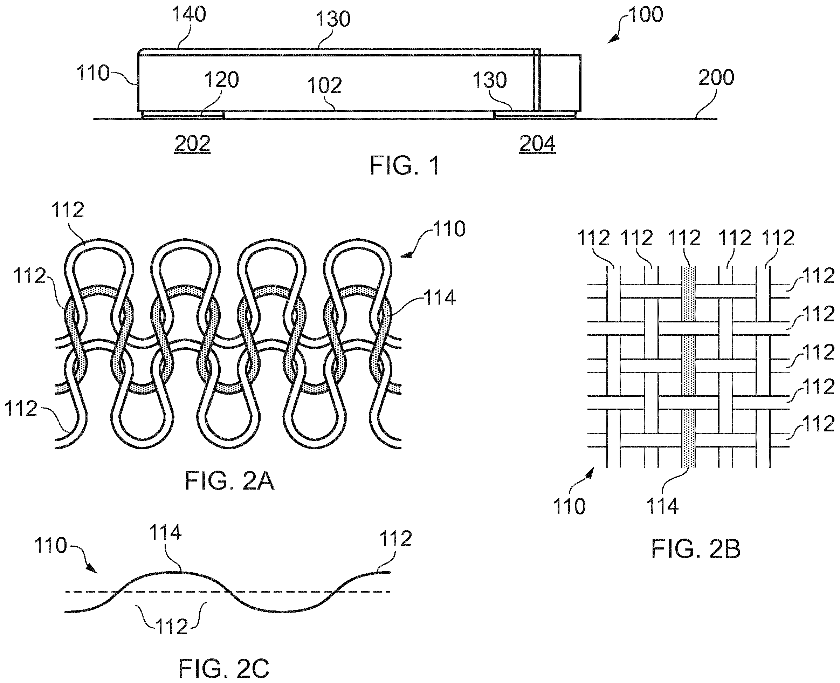

Fig. 1 illustrates an example of an apparatus 100 comprising: a fabric substrate 110; at least one sensing electrode 120 at an interior surface 102 of the fabric substrate 110 for placement adjacent a first portion 202 of a subject's body 200; at least one reference electrode 130 at the interior surface 102 of the fabric substrate 110 for placement adjacent a second portion 204 of the subject's body 200; and a first conductive interconnect 130 connecting to the at least one reference electrode 130, wherein the first conductive interconnect 130 is arranged and positioned on a portion of the fabric substrate 110 other than the interior surface 102 of the fabric substrate 110 to provide at least part of a shield 140 to the at least one sensing electrode 120.

The apparatus 100 has the advantage that it is comfortable and flexible in the long term because it comprises a fabric substrate 110.

The sensing electrode is placed adjacent a portion of a subject's body in use to sense a bio signal from the subject's body. This may, for example, be useful in impedance tomography, sensing of electrocardiograms (ECG), arterial pulse sensing or in other measurements of electrical signals and/or impedance.

Examples of fabric substrates 110 are illustrated in Figs. 2A, 2B and 2C. In each of the examples a fabric substrate 110 comprises filaments 112. Fig. 2A illustrates a fabric substrate 110 that is knitted. Fig. 2B illustrates an example of a fabric substrate 110 that is woven. Fig. 2C illustrates an example of a fabric substrate 110 with a stitched filament 112. In these examples, a single layer of fabric is illustrated. The fabric substrate 110 may be a single layer substrate or a multi-layer substrate.

The term 'filament' is used in this document to describe a material that can be used to create fabric (synonymous with textile, cloth). It is elongate and has a small thickness and/or cross-sectional area (e.g. less than 4 mm²). It may be made of any suitable flexible material and manufactured in any suitable way. It may also be described as a thread or yarn. A filament 112 may be a single filament or a composite of intertwined filaments.

A filament 112 extends in a longitudinal direction (a longitudinal filament) and contacts adjacent filaments 112 at distances along the longitudinal direction to create a fabric

In a knitted fabric (Fig 2A), the longitudinal filament 112 extends in the longitudinal direction in a series of transverse loops (a row). The longitudinal filament 112 contacts the adjacent parallel longitudinal filaments 112 (adjacent rows) via the loops which are at distances along the longitudinal direction. The adjacent rows are inter-looped by having each loop of one row passing through one adjacent loop in one of the adjacent rows.

In a woven fabric (Fig 2B), the longitudinal filament 112 extends in the longitudinal direction without loops (a row). The longitudinal filament 112 contacts filaments 112 extending in the transverse direction (transverse filaments), perpendicular to the longitudinal direction. The transvers filaments 112 are spaced at distances along the longitudinal direction, and they alternately overlie and underlie the longitudinal filaments 112. This creates an interlaced structure of perpendicular warp and weft filaments 112.

In a felted fabric (not illustrated), the longitudinal filament 112 is pressed together with other filaments.

Filaments 112 may thus be used to form the fabric substrate 110. Filaments 112 may also be added to augment the fabric substrate 110 by being stitched (e.g. inter-looped or inter-weaved or embroidered) into a fabric substrate 110 (Fig 2C). Where the fabric substrate 110 is multi-layered each fabric layer may be separately augmented with one or more filaments by stitching. It is thus possible to create three-dimensional, multi-layer textile substrates 110.

In a conductive fabric, one of more of the filaments 110 are electrically conductive for all or part of their length. Other filaments 110 may provide electrical insulation/isolation for the conductive filament.

A fabric conductive-interconnect 114 is that portion of a filament 112 that is electrically conductive and provides an electric current path. The filament 112 may be electrically conductive for all or part of its length. Other non-conductive filaments/portions may provide electrical insulation/isolation for the fabric conductive-interconnect. The fabric conductive-interconnect 114 may be part of the fabric substrate 110, for example a longitudinal filament, that is looped in a knitted fabric (Fig 2A) or a warp or weft in woven fabric (Fig 2B) or an addition to the fabric substrate 110, for example, by stitching (Fig 2C).

A fabric electrode 116 is a conductive portion of fabric formed from multiple fabric conductive-interconnects 114.

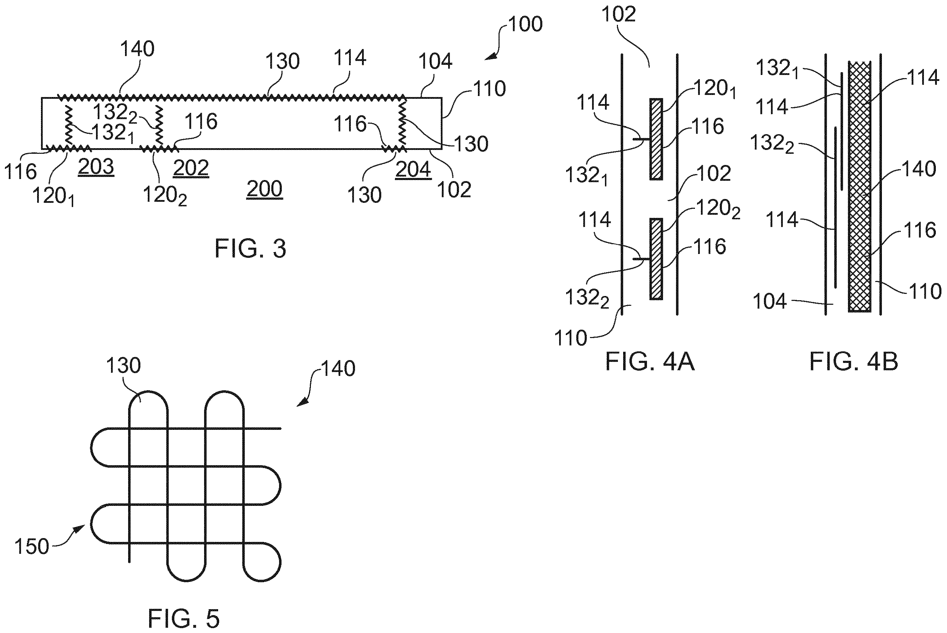

Fig. 3 illustrates an example of the apparatus 100 similar to the apparatus 100 illustrated in Fig. 1, and similar references are used for similar features.

The apparatus 100 comprises: a fabric substrate 110 comprising filaments 112; at least one sensing electrode 120 at an interior surface 102 of the fabric substrate 110 for placement adjacent a first portion 202 of the subject's body 200; at least one reference electrode 130 at the interior surface 102 of the fabric substrate 110 for placement adjacent a second portion 204 of the subject's body 200 and a first conductive interconnect 130 connecting to the at least one reference electrode 130.

The first conductive interconnect 130 is arranged and positioned on a portion of the fabric substrate 110 other than the interior surface 102 of the fabric substrate 110 to provide at least part of a shield 140 to the at least one sensing electrode 120.

The fabric substrate 110 may be a fabric substrate as previously described.

In this example, the first conductive interconnect 130 is a fabric conductive interconnect 114. In this example the first conductive interconnect 130 is formed from a filament 112 of the fabric substrate 110 at a first exterior surface 104 of the fabric substrate 110 that is not the interior surface 102 of the fabric substrate 110.

In this example, the at least one sensing electrode 130 is a fabric electrode 116. In this example, the at least one sensing electrode 130 is formed from one or more filaments 112 of the fabric substrate 110 at the interior surface 102 of the fabric substrate 110.

Also, in this example the at least one reference electrode 130 is a fabric electrode 116. In this example the at least one reference electrode 130 is formed from one or more filaments 112 of the fabric substrate 110 at the interior surface 102 of the fabric substrate 110.

In this example there is a second conductive interconnect 132 connecting to the at least one sensing electrode 120. In this example, the at least one second conductive electrode 132 is a fabric conductive interconnect 114. In this example, the at least one second conductive electrode 132 is formed from a filament of the fabric substrate 110 at a first surface 104 of the fabric substrate 110 that is not an interior surface 102 of the fabric substrate.

As previously described, the fabric conductive interconnects 114 may each be an integral part of the fabric substrate 110 as illustrated in Figs. 2A and 2B or may be an addition to the fabric substrate 110 as illustrated in Fig. 2C.

Fig. 4A illustrates a plan view of an example of the interior surface 102 of the fabric substrate 110 illustrated in Fig 3 and Fig. 4B illustrates a plan view of the exterior surface 104 of the fabric substrate 110 illustrated in Fig 3.

It can be seen that the first conductive interconnect 130 extends along the exterior surface 104 of the fabric substrate 110 and is not present on the interior surface 102. The first conductive interconnect 130 forms shield 140.

It can be seen that the second conductive interconnect 132 extends from the sensing electrode 120 on the interior surface 102, through the thickness of the fabric substrate 110 to the exterior surface 104 of the fabric substrate 110. The second conductive interconnect 132 then extends along the exterior surface 104 of the fabric substrate 110.

In the example illustrated there are multiple sensing electrodes 120 (e.g. 120₁, 120₂...) illustrated and multiple second conductive interconnects 132 (e.g. 132₁, 132₂...) each connecting to a respective one of the multiple sensing electrodes 120. As illustrated in Fig. 4B, both of the second conductive interconnects 132 extend along the exterior surface 104 of the fabric substrate 110 but are separate and independent.

It will therefore be appreciated that the apparatus 100 comprises one or more sensing electrodes 120 that may be positioned at any desired location on the interior surface 102 of the fabric substrate 110 either during the manufacture of the fabric substrate (Figs. 2A, 2B) or added after manufacture (Fig. 2C). Likewise, the reference electrode 130 can also be positioned at an any desired location on the exterior surface 104 of the fabric substrate 110 either during the manufacture of the fabric substrate (Figs. 2A, 2B) or added after manufacture (Fig. 2C).

Each of the conductive interconnects 130, 132 may be a fabric conductive interconnect 114 formed from a filament 112 of the fabric substrate 110. Each of the sensing electrodes 120 may be a fabric electrode 116. The reference electrode 130 may be a fabric electrode 116.

The use of fabric conductive interconnects 114 and fabric electrodes 116 results in a wholly fabric apparatus 100. It avoids the need for laminating and gluing or for solid structures which may make the fabric substrate 110 stiff and uncomfortable to wear.

As illustrated in Figs. 4A and 4B, the apparatus 100 extends over a first area. The conductive interconnects 130, 132 and electrodes 120, 130 are flexible over this first area providing and maintaining flexibility of the apparatus 100 over the first area. This flexibility is maintained and enhanced by, for example, the use of fabric conductive interconnects 114, fabric electrodes 116, and flexible circuitry and by not using stiff laminated conductors or circuitry.

The shield 140 shields the sensing electrode 120, or if there are more than one sensing electrode 120 it may shield one, or some, or all of the sensing electrodes 120. This can be understood from Fig. 4B. The first conductive interconnect 130, as illustrated in Fig. 4B, extends over the exterior surface 104, overlying the sensing electrodes 120 on the interior surface 102 and provides a shield 140 to the sensing electrodes 120. In this example the width of the first conductive interconnect 130 is wider than the width of the sensing electrodes 120 such that the first conductive interconnect entirely overlies the sensing electrodes 120.

The first conductive interconnect 130 shields the at least one sensing electrode 120 from the effects of electric fields for example those caused by electrostatic charges and, in particular, electric charges arising from the triboelectric effect. The triboelectric effect describes a contact electric field arising from frictional contact between two materials. One of these materials may, for example, be the fabric substrate 110. One of the material may, for example, be the body 200 of the subject or other fabric or clothing worn by the subject, if any.

In order to provide a shield 140, the first conductive interconnect 130 need not form a continuous conductive structure. It may, for example, form a mesh structure 150 as illustrated in Fig. 5. In this example, the first conductive interconnect 130 forms a shield 140 in the form of a Faraday cage or Faraday shield that covers the at least one sensing electrode 120.

The first conductive interconnect 130 provides a shield for triboelectric charging 140.

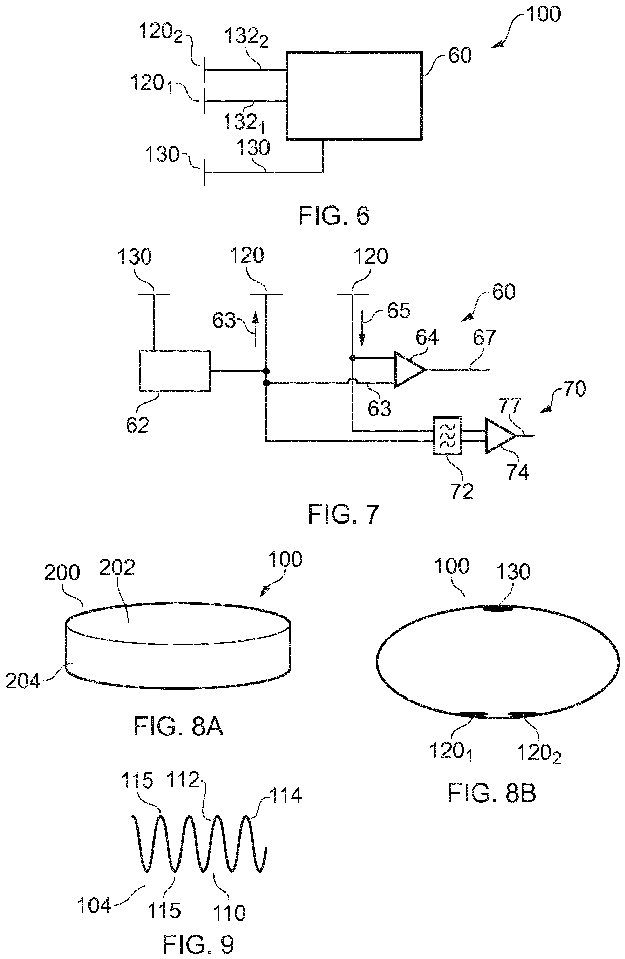

Fig. 6 illustrates an example of the apparatus 100 comprising additional circuitry 60. The circuitry 60 is interconnected to the one or more sensing electrodes 120 (e.g. 120₁, 120₂ ...) by one or more second interconnects 132 (e.g. 132₁, 132₂ ...) and is interconnected to the reference electrode 130 via the first interconnect 130. The reference electrode 130, sensing electrode(s) 120 and interconnects 130, 132 may for example be as described in any of the preceding description.

The reference electrode 130 provides a low-input impedance discharge path for static charges for example those generated by the triboelectric effect. In one example, the first interconnect 130 is a passive conductive path to ground. In another example, the reference electrode 130 is driven, via the first interconnect 130, by a voltage signal to create a virtual ground.

In the example illustrated, the apparatus 100 comprises a first sensing electrode 120, at a position on an interior surface 102 of the fabric substrate 110 for placement adjacent a portion 202 of the subject's body and a second sensing electrode 220₂ at a different position of the interior surface 102 of the fabric substrate 110 for placement adjacent a different portion 203 of the subject's body 200, as illustrated in Fig. 4A.

In this example, but not necessarily all examples, the circuitry 60 may be configured to provide a common-mode voltage to the reference electrode 130. For example, the common-mode voltage between the sensing electrodes 120 (e.g. 120₁, 120₂) may be obtained, and inverted and then driven back to the subject body 200 via the reference electrode 130. This makes the reference electrode 130 active, but still provides a low input impedance for the triboelectric charges to be discharged.

The circuitry 60 may be flexible circuitry such that it does not compromise or overly compromise the flexibility of the apparatus 100.

The apparatus 100 is therefore flexible because the fabric substrate 110 is flexible and the other components of the apparatus 100 are also flexible including the circuitry 60.

Fig. 7 illustrates an example of the apparatus 100, similar to that illustrated in Fig 6, in which the circuitry 60 additionally or alternatively comprises electrocardiograph (ECG) circuitry 64 and signal-source circuitry 62 and signal-receiver circuitry 70.

The ECG circuitry 64 comprises an op-amp that has one input connected to one sensing electrode 120₁ and the other input connected to another sensing electrode 120₂. The op-amp produces, at its output, a signal 67 representative of the voltage between the sensing electrodes 120₁, 120₂. The signal 67 is an ECG signal (a signal dependent upon the polarization and de-polarization of the subject's heart) when the sensing electrodes 120₁, 120₂ are correctly positioned on the subject's body 200.

The signal-source circuitry 62 is configured to provide a time varying signal 63 as an input signal to one of the sensing electrodes 120₁. This signal travels through the subject's body 200 and is received at the other of the sensing electrodes 120₂ as a received time varying signal 65.

The frequency of the time varying signal 63 is preferably outside the frequency band of the signal of interest. For example, if it is desired to measure ECG using the sensing electrodes 120, it is desirable to have the frequency of the input signal 63 outside the ECG band (for example, the input signal 63 has a frequency in the range 4 kHz to 1 MHz). This input signal 63 is modulated by the impedance between the signal-source circuitry 62 and the signal-receiver circuitry 70.

Signal-receiver circuitry 70 measures changes in the received time-varying signal 65 arising from changes in electrical impedance between the signal-source circuitry 62 and the signal-receiver circuitry 70.

The signal-receiver circuitry 70 may comprise a high pass filter 72, so that it represents a high impedance to the ECG signal.

After filtering, the signal-receiver circuitry 70 may comprise an op-amp 74 that has one input coupled to the sensing electrode 120₁ through the filter 70 and the other input connected to the sensing electrode 120₂ through the filter 70. The op-amp 74 produces, at its output, a signal 77 representative of the impedance from the signal-source circuitry 62, via the sensing electrodes 120₁, 120₂ to the signal-receiver circuitry 70.

Where variations in the impedance from the signal-source circuitry 62, via the sensing electrodes 120₁, 120₂ to the signal-receiver circuitry 70 is dominated by variation in impedance of the body 200, the apparatus 100 is configured to determine a variable internal impedance of the body 200. This variable impedance may for example be used for impedance tomography or arise from changing fluid in the thorax, for example liquid in the lung.

In some examples, the apparatus 100 is designed so that the impedance from the signal-source circuitry 62, via the sensing electrodes 120₁, 120₂ to the signal-receiver circuitry 70 is dominated by variation in an impedance other than that of the body 200.

For example, the electric path between the signal-source circuitry 62 and the signal-receiver circuitry 70 may comprise the at least one fabric conductor interconnect 114 configured to change impedance with a change in tension during use. In this example, the apparatus 100 is capable of detecting a change in resistance arising from the tensioning or a change in tensioning of the fabric conductive-interconnect 114. Such a change may be caused by breathing of the subject.

Fig. 8A illustrates a perspective view of the apparatus 100 configured as a strap 200, to be placed around a body portion of the subject. In some, but not necessarily all examples, the strap is a chest-strap designed to be placed around the thorax of the subject. Fig. 8B illustrates a view of the apparatus 100 from a plan view.

The apparatus 100 may be configured as described with reference to Fig 7 to provide an ECG signal 67 and a signal 77 dependent upon breathing, for example.

The dimensions of the strap 200 along its length is shorter than the chest circumference of the user so that when the chest strap 200 is in place, it is under tension. There may be a buckle or clasp 202 that allows the strap 200 to be closed around the thorax of the user. There may also be a tensioning means 204 that allows the effective length of the strap to be varied and thereby control the tension within the
strap 200 when it is in use. The apparatus 100 is therefore configured to tension the fabric substrate 110 in use.

As illustrated in Fig. 8B, the chest strap 200 is configured to position the at least one sensing electrode 120 in contact with a front or side portion of the subject's thorax and to position the at least one reference electrode 130 in contact with a rear portion of the subject's thorax. In this example the chest strap 200 is configured to maximize a distance of separation between the at least one sensing electrode 120 and the at least one reference electrode 130 when measured along the exterior of the subject's thorax.

The apparatus 100 is configured to tension the fabric structure 110 in use. In some examples, the fabric structure 110 comprises at least one fabric conductive interconnect 114 configured to produce a change in impedance with a change in tension during use. Such a change in impedance may be dependent upon breathing.

The fabric conductive interconnect 114 may be positioned in the electric path between the signal-source circuitry 62 and the signal-receiver circuitry 70.

Fig. 9 illustrates an example of a suitable fabric conductive interconnect 114 configured to cause a change in impedance with a change in tension during use. In this example, the fabric conductive interconnect 114 comprises a resiliently extendable filament 112 stitched into the fabric substrate 110. By controlling the length of the filament 112 it is possible to control the ratio of the impedance in the body to the ratio of any resistance change in the resiliently extendable filament 114.

In the example illustrated the fabric conductive interconnect 114 comprises a resiliently extendible filament 112 stitched into the fabric structure substrate 110 so that it extends along a surface 104 of the fabric substrate 104 in a series of alternating curves 115. This gives it a switch-back shape.

Any of the apparatus 100 described may be in the form of clothing, where the fabric substrate 110 is part of the clothing.

In some but not necessarily all examples, the apparatus 100 is configured to communicate data from the apparatus 100 with or without local storage of the data in a memory at the apparatus and with or without local processing of the data by circuitry or processors at the apparatus 100.

The data may, for example, be measurement data from one or more sensing electrodes 120 or data produced by the processing of measurement data from one or more sensing electrodes 120, such as, for example, an ECG signal 57 and/or an impedance signal 77.

The data may be stored in processed or unprocessed format remotely at one or more devices. The data may be stored in The Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links such a E-UTRANS. The apparatus may comprise a communications interface such as, for example, a radio transceiver for communication of data.

The apparatus 100 may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train an acyclic machine learning network such as a multilayer neural network or may be used as a query input to a machine learning network, which provides a response.

The processing of the data, whether local or remote, may produce an output. The output may be communicated to the apparatus 100 where it may produce an output sensible to the subject such as an audio output, visual output or haptic output.

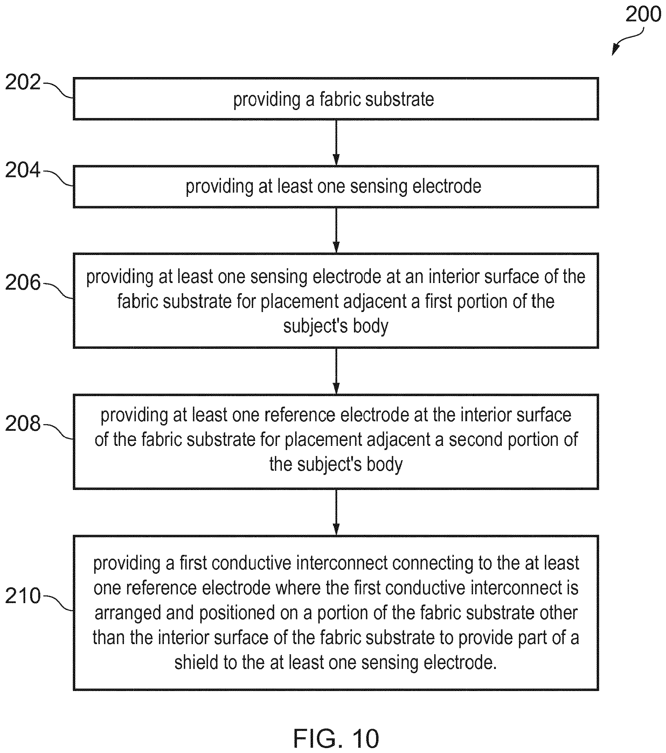

It will be appreciated from the foregoing that the preceding description describes examples of a method 200, as illustrated in Fig 10. The method 200 comprises:
at block 202, providing a fabric substrate 110;
at block 204, providing at least one sensing electrode 120;
at block 206, providing at least one sensing electrode 120 at an interior surface 102 of the fabric substrate 110 for placement adjacent a first portion 204 of the subject's body;
at block 208, providing at least one reference electrode 130 at the interior surface 102 of the fabric substrate 110 for placement adjacent a second portion 202 of the subject's body 200; and
at block 210, providing a first conductive interconnect 130 connecting to the at least one reference electrode 130 where the first conductive interconnect 130 is arranged and positioned on a portion of the fabric substrate 110 other than the interior surface of the fabric substrate 110 to provide part of a shield to the at least one sensing electrode 130.

As used in this application, the term 'circuitry' refers to all of the following:
(a) hardware-only circuit implementations (such as implementations in only analog and/or digital circuitry) and
(b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a mobile phone or server, to perform various functions and
(c) to circuits, such as a microprocessor(s) or a portion of a microprocessor(s), that require software or firmware for operation, even if the software or firmware is not physically present.
This definition of 'circuitry' applies to all uses of this term in this application, including in any claims. As a further example, as used in this application, the term "circuitry"
would also cover an implementation of merely a processor (or multiple processors) or portion of a processor and its (or their) accompanying software and/or firmware. The term "circuitry" would also cover, for example and if applicable to the particular claim element, a baseband integrated circuit or applications processor integrated circuit for a mobile phone or a similar integrated circuit in a server, a cellular network device, or other network device.

Where a structural feature has been described, it may be replaced by means for performing one or more of the functions of the structural feature whether that function or those functions are explicitly or implicitly described.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

## Claims

1. An apparatus (100) comprising:
a fabric substrate (110);
at least one sensing electrode (120) at an interior surface (102) of the fabric substrate (110) for placement adjacent a first portion (202) of a subject's body (200);
at least one reference electrode (130) at the interior surface (102) of the fabric substrate (110) for placement adjacent a second portion (204) of a subject's body (200); and
a first conductive interconnect connecting to the at least one reference electrode (130) wherein the
first conductive interconnect is arranged and positioned to provide part of a shield to the at least one sensing electrode (120) **characterized in that** the first conductive interconnect is positioned on a portion of the fabric substrate (110) other than the interior surface (102) of the fabric substrate (110).

2. An apparatus (100) as claimed in claim 1, wherein the first conductive interconnect is a fabric conductive-interconnect at a first surface of the fabric substrate (110).

3. An apparatus (100) as claimed in claim 1 or 2,
wherein the at least one sensing electrode (120) is a fabric electrode at a second surface, the interior surface (102), of the fabric substrate (110) and/or
wherein the at least one reference electrode (130) is a fabric electrode at a second surface, the interior surface (102), of the fabric substrate (110).

4. An apparatus (100) as claimed in any preceding claim, comprising at least one second conductive interconnect connecting to the at least one sensing electrode (120), wherein the at least one second conductive interconnect is a fabric conductive-interconnect.

5. An apparatus (100) as claimed in any preceding claim, comprising flexible circuitry operatively interconnected to the at least one sensing electrode (120) and the at least one reference electrode (130) for producing a sensing signal.

6. An apparatus (100) as claimed in any preceding claim configured as a chest strap configured to position the at least one sensing electrode (120) in contact with a front or side portion of a subject's thorax and to position the at least one reference electrode (130) in contact with a rear portion of a subject's thorax.

7. An apparatus (100) as claimed in claim 6, configured to maximize a distance of separation between the at least one sensing electrode (120) and the at least one reference electrode (130).

8. An apparatus (100) as claimed in any preceding claim,
wherein the at least one sensing electrode (120) comprises a first sensing electrode (120₁) at a position on an interior surface (102) of the fabric substrate (110) for placement adjacent a portion of a subject's body; and
a second sensing electrode (120₂) at a different position on the interior surface (102) of the fabric substrate (110) for placement adjacent a different portion of a subject's body;
the apparatus (100) comprising circuitry configured to provide a common mode voltage at the reference electrode (130).

9. An apparatus (100) as claimed in any preceding claim,
wherein the at least one sensing electrode (120) comprises a first sensing electrode (120₁) at a position on an interior surface (102) of the fabric substrate (110) for placement adjacent a portion of a subject's body; and
a second sensing electrode (120₂) at a different position on the interior surface (102) of the fabric substrate (110) for placement adjacent a different portion of a subject's body;
the apparatus (100) comprising ECG circuitry configured to provide an ECG signal from the first sensing electrode (120₁) and the second sensing electrode (120₂);
input circuitry configured to apply a time-varying input signal at the first sensing electrode (120₁); and
output circuitry configured to detect a time-varying output signal at the second sensing electrode (120₂).

10. An apparatus (100) as claimed in any preceding claim configured to tension the fabric substrate (110) in use, the fabric substrate (110) comprising at least a fabric conductive-interconnect configured to resiliently change impedance with a change in tension during use.

11. An apparatus (100) as claimed in claim 10, when dependent upon claim 9, wherein an electric path between input circuitry and output circuitry comprises the at least one fabric conductive-interconnect configured to resiliently change impedance with a change in tension during use.

12. An apparatus (100) as claimed in claim 10 or 11, wherein the fabric conductive-interconnect comprises a resiliently extendible filament stitched into the fabric substrate (110).

13. An apparatus (100) as claimed in claim 12, wherein the resiliently extendible filament is stitched into the fabric substrate (110) so that it extends along a surface of the fabric substrate (110) in a series of alternating curves.

14. An apparatus (100) as claimed in any preceding claim in the form of clothing, wherein the fabric substrate (110) is part of the clothing.

15. A method comprising:
providing a fabric substrate (110);
providing at least one sensing electrode (120) at an interior surface (102) of the fabric substrate (110) for placement adjacent a first portion (202) of a subject's body (200);
providing at least one reference electrode (130) at the interior surface (102) of the fabric substrate (110) for placement adjacent a second portion (204) of a subject's body (200); and
providing a first conductive interconnect connecting to the at least one reference electrode (130)
wherein the
first conductive interconnect is arranged and positioned on a portion of the fabric substrate (110) other than the interior surface (102) of the fabric substrate (110) to provide part of a shield to the at least one sensing electrode (120).

## Patentansprüche

1. Vorrichtung (100), die Folgendes umfasst:
ein Gewebesubstrat (110);
mindestens eine Erfassungselektrode (120) auf einer Innenfläche (102) des Gewebesubstrats (110) zur Platzierung neben einem ersten Abschnitt (202) eines Körpers (200) eines Patienten;
mindestens eine Referenzelektrode (130) auf der Innenfläche (102) des Gewebesubstrats (110) zur Platzierung neben einem zweiten Abschnitt (204) eines Körpers (200) eines Patienten; und
eine erste leitfähige Zwischenverbindung, die mit der mindestens einen Referenzelektrode (130) verbunden ist,
wobei die erste leitfähige Zwischenverbindung angeordnet und positioniert ist, um einen Teil einer Abschirmung gegenüber der mindestens einen Erfassungselektrode (120) bereitzustellen
**dadurch gekennzeichnet, dass** die erste leitfähige Zwischenverbindung auf einem anderen Abschnitt des Gewebesubstrats (110) als der Innenfläche (102) des Gewebesubstrats (110) positioniert ist.

2. Vorrichtung (100) nach Anspruch 1, wobei die erste leitfähige Zwischenverbindung eine leitfähige Gewebezwischenverbindung auf einer ersten Fläche des Gewebesubstrats (110) ist.

3. Vorrichtung (100) nach Anspruch 1 oder 2,
wobei die mindestens eine Erfassungselektrode (120) eine Gewebeelektrode auf einer zweiten Fläche, der Innenfläche (102), des Gewebesubstrats (110) ist und/oder
wobei die mindestens eine Referenzelektrode (130) eine Gewebeelektrode auf einer zweiten Fläche, der Innenfläche (102), des Gewebesubstrats (110) ist.

4. Vorrichtung (100) nach einem der vorhergehenden Ansprüche, die mindestens eine zweite leitfähige Zwischenverbindung umfasst, die mit der mindestens einen Erfassungselektrode (120) verbunden ist, wobei die mindestens eine zweite leitfähige Zwischenverbindung eine leitfähige Gewebezwischenverbindung ist.

5. Vorrichtung (100) nach einem der vorhergehenden Ansprüche, die eine flexible Schaltung umfasst, die zum Produzieren eines Erfassungssignals mit der mindestens einen Erfassungselektrode (120) und der mindestens einen Referenzelektrode (130) wirkmäßig zusammengeschaltet ist.

6. Vorrichtung (100) nach einem der vorhergehenden Ansprüche, die als ein Brustgurt ausgelegt ist, der dazu ausgelegt ist, die mindestens eine Erfassungselektrode (120) mit einem vorderen oder einem seitlichen Abschnitt eines Thorax eines Patienten in Kontakt zu bringen und die mindestens eine Referenzelektrode (130) mit einem hinteren Abschnitt eines Thorax eines Patienten in Kontakt zu bringen.

7. Vorrichtung (100) nach Anspruch 6, die dazu ausgelegt ist, einen Abstand der Trennung zwischen der mindestens einen Erfassungselektrode (120) und der mindestens einen Referenzelektrode (130) zu maximieren.

8. Vorrichtung (100) nach einem der vorhergehenden Ansprüche,
wobei die mindestens eine Erfassungselektrode (120) eine erste Erfassungselektrode (120₁) in einer Position auf einer Innenfläche (102) des Gewebesubstrats (110) zur Platzierung neben einem Abschnitt eines Körpers eines Patienten umfasst; und
eine zweite Erfassungselektrode (120₂) in einer anderen Position auf der Innenfläche (102) des Gewebesubstrats (110) zur Platzierung neben einem anderen Abschnitt eines Körpers eines Patienten;
wobei die Vorrichtung (100) eine Schaltung umfasst, die dazu ausgelegt ist, eine Gleichtaktspannung an der Referenzelektrode (130) bereitzustellen.

9. Vorrichtung (100) nach einem der vorhergehenden Ansprüche,
wobei die mindestens eine Erfassungselektrode (120) eine erste Erfassungselektrode (120₁) in einer Position auf einer Innenfläche (102) des Gewebesubstrats (110) zur Platzierung neben einem Abschnitt eines Körpers eines Patienten umfasst; und
eine zweite Erfassungselektrode (120₂) in einer anderen Position auf der Innenfläche (102) des Gewebesubstrats (110) zur Platzierung neben einem anderen Abschnitt eines Körpers eines Patienten;
wobei die Vorrichtung (100) eine EKG-Schaltung umfasst, die dazu ausgelegt ist, ein EKG-Signal von der ersten Erfassungselektrode (120₁) und der zweiten Erfassungselektrode (120₂) bereitzustellen;
Eingangsschaltung, die dazu ausgelegt ist, an die erste Erfassungselektrode (120₁) ein zeitlich variierendes Eingangssignal anzulegen; und
Ausgangsschaltung, die dazu ausgelegt ist, an der zweiten Erfassungselektrode (120₂) ein zeitlich variierendes Ausgangssignal zu detektieren.

10. Vorrichtung (100) nach einem der vorhergehenden Ansprüche, die dazu ausgelegt ist, das Gewebesubstrat (110) im Gebrauch zu spannen, wobei das Gewebesubstrat (110) mindestens eine leitfähige Gewebezwischenverbindung umfasst, die dazu ausgelegt ist, während des Gebrauchs bei einer Änderung der Spannung eine Impedanz resilient zu ändern.

11. Vorrichtung (100) nach Anspruch 10, sofern von Anspruch 9 abhängig, wobei der elektrische Pfad zwischen Eingangsschaltung und Ausgangsschaltung die mindestens eine leitfähige Gewebezwischenverbindung umfasst, die dazu ausgelegt ist, während des Gebrauchs bei einer Änderung der Spannung eine Impedanz resilient zu ändern.

12. Vorrichtung (100) nach Anspruch 10 oder 11, wobei die leitfähige Gewebezwischenverbindung einen resilient dehnbaren Faden umfasst, der in das Gewebesubstrat (110) eingenäht ist.

13. Vorrichtung (100) nach Anspruch 12, wobei der resilient dehnbare Faden derart in das Gewebesubstrat (110) eingenäht ist, dass er sich entlang einer Fläche des Gewebesubstrats (110) in einer Reihe von abwechselnden Kurven dehnt.

14. Vorrichtung (100) nach einem der vorhergehenden Ansprüche in Form eines Kleidungsstücks, wobei das Gewebesubstrat (110) Teil des Kleidungsstücks ist.

15. Verfahren, das Folgendes umfasst:
Bereitstellen eines Gewebesubstrats (110);
Bereitstellen von mindestens einer Erfassungselektrode (120) auf einer Innenfläche (102) des Gewebesubstrats (110) zur Platzierung neben einem ersten Abschnitt (202) eines Körpers (200) eines Patienten;
Bereitstellen von mindestens einer Referenzelektrode (130) auf der Innenfläche (102) des Gewebesubstrats (110) zur Platzierung neben einem zweiten Abschnitt (204) eines Körpers (200) eines Patienten; und
Bereitstellen einer ersten leitfähigen Zwischenverbindung, die mit der mindestens einen Referenzelektrode (130) verbunden ist
wobei die erste leitfähige Zwischenverbindung auf einem anderen Abschnitt des Gewebesubstrats (110) als der Innenfläche (102) des Gewebesubstrats (110) angeordnet und positioniert ist, um einen Teil einer Abschirmung gegenüber der mindestens einen Erfassungselektrode (120) bereitzustellen.

## Revendications

1. Appareil (100) comprenant :
un substrat en tissu (110) ;
au moins une électrode de détection (120) sur une surface intérieure (102) du substrat en tissu (110) devant être placée de manière adjacente à une première partie (202) du corps d'un sujet (200) ;
au moins une électrode de référence (130) sur la surface intérieure (102) du substrat en tissu (110) devant être placée de manière adjacente à une deuxième partie (204) du corps d'un sujet (200) ; et
une première interconnexion conductrice se connectant à l'au moins une électrode de référence (130),
dans lequel la première interconnexion conductrice est agencée et positionnée pour fournir une section d'un blindage à l'au moins une électrode de détection (120),
**caractérisé en ce que** la première interconnexion conductrice est positionnée sur une partie du substrat en tissu (110) autre que la surface intérieure (102) du substrat en tissu (110).

2. Appareil (100) selon la revendication 1, dans lequel la première interconnexion conductrice est une interconnexion conductrice en tissu sur une première surface du substrat en tissu (110).

3. Appareil (100) selon la revendication 1 ou 2,
dans lequel l'au moins une électrode de détection (120) est une électrode en tissu sur une deuxième surface, la surface intérieure (102), du substrat en tissu (110) et/ou
dans lequel l'au moins une électrode de référence (130) est une électrode en tissu sur une deuxième surface, la surface intérieure (102), du substrat en tissu (110).

4. Appareil (100) selon l'une des revendications précédentes, comprenant au moins une deuxième interconnexion conductrice se connectant à l'au moins une électrode de détection (120), dans lequel l'au moins une deuxième interconnexion conductrice est une interconnexion conductrice en tissu.

5. Appareil (100) selon l'une des revendications précédentes, comprenant une circuiterie flexible interconnectée de manière opérationnelle à l'au moins une électrode de détection (120) et à l'au moins une électrode de référence (130) pour produire un signal de détection.

6. Appareil (100) selon l'une des revendications précédentes configuré comme une sangle de poitrine configurée pour positionner l'au moins une électrode de détection (120) en contact avec une partie avant ou latérale du thorax d'un sujet, et pour positionner l'au moins une électrode de référence (130) en contact avec une partie arrière du thorax d'un sujet.

7. Appareil (100) selon la revendication 6, configurée pour maximiser une distance de séparation entre l'au moins une électrode de détection (120) et l'au moins une électrode de référence (130).

8. Appareil (100) selon l'une des revendications précédentes,
dans lequel l'au moins une électrode de détection (120) comprend une première électrode de détection (120₁) à une position sur une surface intérieure (102) du substrat en tissu (110) devant être placée de manière adjacente à une partie du corps d'un sujet ; et
une deuxième électrode de détection (120₂) à une position différente sur la surface intérieure (102) du substrat en tissu (110) devant être placée de manière adjacente à une partie différente du corps d'un sujet ;
l'appareil (100) comprenant une circuiterie configurée pour fournir une tension de mode commun à l'électrode de référence (130).

9. Appareil (100) selon l'une des revendications précédentes,
dans lequel l'au moins une électrode de détection (120) comprend une première électrode de détection (120₁) à une position sur une surface intérieure (102) du substrat en tissu (110) devant être placée de manière adjacente à une partie du corps d'un sujet ; et
une deuxième électrode de détection (120₂) à une position différente sur la surface intérieure (102) du substrat en tissu (110) devant être placée de manière adjacente à une partie différente du corps d'un sujet ;
l'appareil (100) comprenant une circuiterie ECG configurée pour fournir un signal ECG à partir de la première électrode de détection (120₁) et de la deuxième électrode de détection (120₂) ;
une circuiterie d'entrée configurée pour appliquer un signal d'entrée variable dans le temps au niveau de la première électrode de détection (120₁) ; et
une circuiterie de sortie configurée pour détecter un signal de sortie variable dans le temps au niveau de la deuxième électrode de détection (120₂).

10. Appareil (100) selon l'une des revendications précédentes configuré pour tendre le substrat en tissu (110) en cours d'utilisation, le substrat en tissu (110) comprenant au moins une interconnexion conductrice en tissu configurée pour changer l'impédance de manière élastique avec un changement de tension en cours d'utilisation.

11. Appareil (100) selon la revendication 10 lorsqu'elle dépend de la revendication 9, dans lequel un chemin électrique entre une circuiterie d'entrée et une circuiterie de sortie comprend l'au moins une interconnexion conductrice en tissu configurée pour changer l'impédance de manière élastique avec un changement de tension en cours d'utilisation.

12. Appareil (100) selon la revendication 10 ou 11, dans lequel l'interconnexion conductrice en tissu comprend un filament extensible élastiquement cousu dans le substrat en tissu (110).

13. Appareil (100) selon la revendication 12, dans lequel le filament extensible élastiquement est cousu dans le substrat en tissu (110) de sorte qu'il s'étende le long d'une surface du substrat en tissu (110) dans une série de courbes alternées.

14. Appareil (100) selon l'une des revendications précédentes sous forme de vêtement, dans lequel le substrat en tissu (110) fait partie du vêtement.

15. Procédé comprenant :
la fourniture d'un substrat en tissu (110) ;
la fourniture d'au moins une électrode de détection (120) sur une surface intérieure (102) du substrat en tissu (110) devant être placée de manière adjacente à une première partie (202) du corps d'un sujet (200) ;
la fourniture d'au moins une électrode de référence (130) sur la surface intérieure (102) du substrat en tissu (110) devant être placée de manière adjacente à une deuxième partie (204) du corps d'un sujet (200) ; et
la fourniture d'une première interconnexion conductrice se connectant à l'au moins une électrode de référence (130),
dans lequel la première interconnexion conductrice est agencée et positionnée sur une partie du substrat en tissu (110) autre que la surface intérieure (102) du substrat en tissu (110) pour fournir une section d'un blindage à l'au moins une électrode de détection (120).
